# EUROPÄISCHE PATENTANMELDUNG

(11) **EP 2 617 453 A1**
(43) Veröffentlichungstag der Anmeldung: **24.07.2013**
(21) Anmeldenummer: 12192909.5
(22) Anmeldetag: 16.11.2012
(51) Int. Cl.: A61M 25/01, A61M 25/10, A61B 5/00, A61B 19/00, A61M 25/00

(54) **Katheter und Katheteranordnung**

(30) Priorität: 19.01.2012 US 201261588195 P
(71) Anmelder: VascoMed GmbH, 79589 Binzen (DE)
(72) Erfinder: Knorr, Stefan, 10119 Berlin (DE); Weiss, Ingo, 12435 Berlin (DE); Fandrey, Stephan, 8910 Affoltern am Albis (DE); Geistert, Wolfgang, 79618 Rheinfelden (DE)
(74) Vertreter: Lindner-Vogt, Karin L.

(57) **Zusammenfassung**

Katheter (10) mit einem langgestreckten Katheterkörper (13), der in Bezug auf eine Gebrauchslage ein distales (10d) und ein proximales Ende (10p) hat, wobei am distalen Ende ein schwamm- oder kissenartiger elastischer Deformationskörper (11) angeordnet ist, welcher, insbesondere elektrisch, mechanisch oder optisch wirkende, Messmittel oder einen Messanschluss zur Erfassung einer auf ihn ausgeübten Andruckkraft aufweist.

## Beschreibung

Die Erfindung betrifft einen Katheter mit einem langgestreckten Katheterkörper, der in Bezug auf eine Gebrauchslage ein distales und ein proximales Ende hat. Sie betrifft des Weiteren eine Katheteranordnung mit einem solchen Katheter und einer daran angeschlossenen Messeinrichtung.

In der medizinischen Praxis ist eine Vielzahl verschieden konstruierter Katheter oder katheterähnlicher Einrichtungen (z.B. Elektrodenleitungen) bekannt und in massenhaftem Gebrauch. Sie werden teilweise von erfahrenen Spezialisten, teilweise aber auch von Ärzten oder auch medizinischem Personal ohne spezielle Kenntnisse und Erfahrung angewendet. Schädigungen oder Beeinträchtigungen des Patienten müssen gleichwohl zuverlässig ausgeschlossen werden.

Beim Einsatz bekannter Katheter mit einer Kunststoff- oder Metallspitze trifft eine Perforationsgefahr auf. Um die Flächenpressung und damit die Perforationsgefahr gering zu halten, müssen bei der Steifheit des Katheterschaftes und der Katheterspitze Kompromisse eingegangen werden. Diese Kompromisse schränken u. U. die Manövrierfähigkeit und die Lagestabilität des Katheters ein.

Der Erfindung liegt daher die Aufgabe zugrunde, einen verbesserten Katheter bereitzustellen, der ohne die Gefahr von Schädigungen oder Beeinträchtigungen des Patienten auch von weniger Geübten eingesetzt werden kann, dessen Konstruktion dennoch ohne weiteres den im klinischen Einsatz bestehenden Anforderungen gerecht wird und dessen Gebrauchswert insgesamt gesteigert ist.

Diese Aufgabe wird durch einen Katheter mit den Merkmalen des Anspruchs 1 gelöst. Mit der Erfindung wird des Weiteren eine Katheteranordnung mit den Merkmalen des Anspruchs 14 bereitgestellt.

Die Erfindung geht von dem Gedanken aus, bekannte Katheteraufbauten durch ein spezielles Perforationsschutzelement zu modifizieren. Sie schließt weiter den Gedanken ein, einen Deformationskörper am distalen Ende des Katheters vorzusehen. Schließlich gehört zur Erfindung der Gedanke, aus dem Deformationskörperverhalten des Deformationskörpers über geeignete Messmittel Daten zu gewinnen, die eine verbesserte Beurteilung der konkreten Einsatzsituation ermöglichen. Im Einsatz des Katheters unterliegt der Deformationskörper beim Andrücken an die Wandung eines Gefäßes oder Hohlorgans einer Druckänderung, und die Druckänderung kann distal oder proximal gewandelt werden. Die beim Andrücken an das Gewebe erfolgende Druckänderung kann zu einer Änderung der elektrischen Eigenschaften des Deformationskörpers (Widerstand, Kapazität,...) führen, welche gemessen und zur Ermittlung der Andruckfläche und/oder Andruckkraft genutzt werden können. Auch eine direkte mechanische Signalübertragung ist möglich.

In zweckmäßigen Ausgestaltungen der Erfindung ist vorgesehen, dass der Deformationskörper einen Kunststoffschaum oder ein Fluid, insbesondere eine Flüssigkeit oder ein Gel, oder eine Füllung aus schüttfähigen Partikeln, enthält. Da der Deformationskörper elastisch reagieren muss, ist bei Befüllung mit einem Fluid ein auf die Oberfläche und Beschaffenheit der Hüllschicht abgestimmter Füllgrad zu beachten.

In einer weiter oben bereits allgemein angesprochenen messtechnischen Ausführung sind in einander gegenüberliegenden Mantelabschnitten des Deformationskörpers Messelektroden mit jeweils einem Messspannungsanschluss vorgesehen. In einer Ausgestaltung weist der Deformationskörper einen Schaum aus einem elektrisch leitfähigen Polymeren oder mit elektrisch leitfähigen, fein verteilten Einlagerungen oder mit leitfähigen Partikeln, die mit einem Dielektrikum beschichtet sind, oder Teilchen aus einer feroelektrischen Folie auf.

In weiteren Ausgestaltungen der Erfindung ist vorgesehen, dass am distalen Ende des Katheterkörpers, starr mit diesem verbunden, ein erstes Messvorrichtungselement und auf der Innenwandung einer Hülle des Deformationskörpers ein zweites, mit dem ersten zusammenwirkendes Messvorrichtungselement vorgesehen ist. Die Hülle des Deformationskörpers ist durch Ihre eigene Elastizität und/oder ihr Zusammenwirken mit einer Füllung und/oder ihr Zusammenwirken mit mindestens einem Federelement, welches die Hülle gegenüber dem Katheterkörper abstützt, bezüglich des Katheterkörpers selbst-rückstellend ausgeführt.

Eine besonders einfache rein mechanische Ausführung der vorgesehenen Messmittel kann so aussehen, dass an einem distalen Ende des Deformationskörpers ein sich bis zum proximalen Ende des Katheters erstreckender, im Katheterkörper verlaufender und frei verschieblicher Messdraht angebracht ist. Die mit einem Stauchen des Deformationskörpers verbundene Verschiebung des steifen Messdrahtes längs des Katheterkörpers wird an dessen proximalen Ende Sicht- und quantitativ erfassbar; eine Kraftmessung erfordert allerdings zusätzliche messtechnische Vorkehrungen. In einer Abwandlung kann die Funktion des Messdrahtes bis zu einem gewissen Grade auch vom Innenschlauch selbst übernommen werden.

In einer weiteren Ausführung ist der Deformationskörper mehrteilig aus mehreren Teilkörpern ausgebildet, wobei die Teilkörper unterschiedliches Deformationsverhalten und/oder separate Messmittel oder -anschlüsse zur Erfassung einer spezifisch auf sie ausgeübten Andruckkraft aufweisen. Hierbei ist insbesondere vorgesehen, dass die Teilkörper optisch oder elektrisch wirkende Messmittel oder -anschlüsse aufweisen.

In einer weiteren Ausführung ist der Katheter ausgebildet als Elektrodenleitung mit mindestens einer auf dem Deformationskörper oder am distalen Ende des Innenschlauches, umgeben vom Deformationskörper, angeordneten Elektrode. Hierbei ist insbesondere die oder mindestens eine Elektrode elastisch deformierbar, insbesondere aus leitfähigem Kunststoff gebildet.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
Fig. 1A bis 1E schematische Darstellungen (Seitenansichten bzw. Querschnittsdarstellungen) einer Ausführungsform des erfindungsgemäßen Katheters im Ausgangszustand bzw. deformierten Zustand des Katheterendes,
Fig. 2A und 2B schematische perspektivische Darstellungen einer weiteren Ausführungsform des Katheters,
Fig. 3A bis 3C schematische perspektivische Darstellungen einer weiteren Ausführungsform des Katheters,
Fig. 4 eine perspektivische Darstellung einer weiteren Ausführungsform des erfindungsgemäßen Katheters,
Fig. 5 eine schematische Darstellung einer Ausführungsform des vorgeschlagenen Katheters als bipolare Elektrodenleitung,
Fig. 6 eine schematische Darstellung einer weiteren Ausführungsform des erfindungsgemäßen Katheters und
Fig. 7A und 7B Prinzipskizzen von Ausführungsformen der erfindungsgemäßen Katheteranordnung.

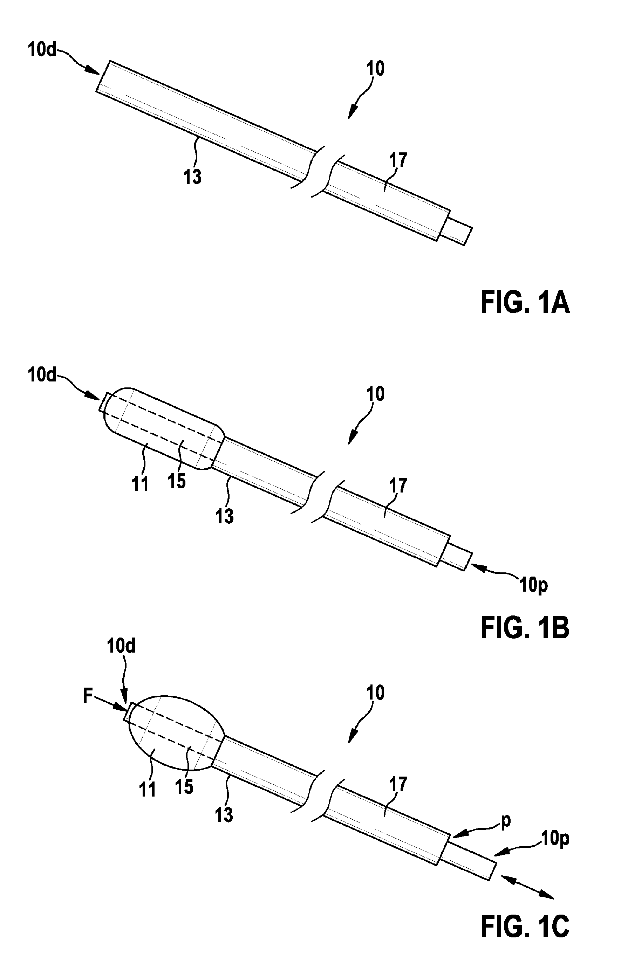

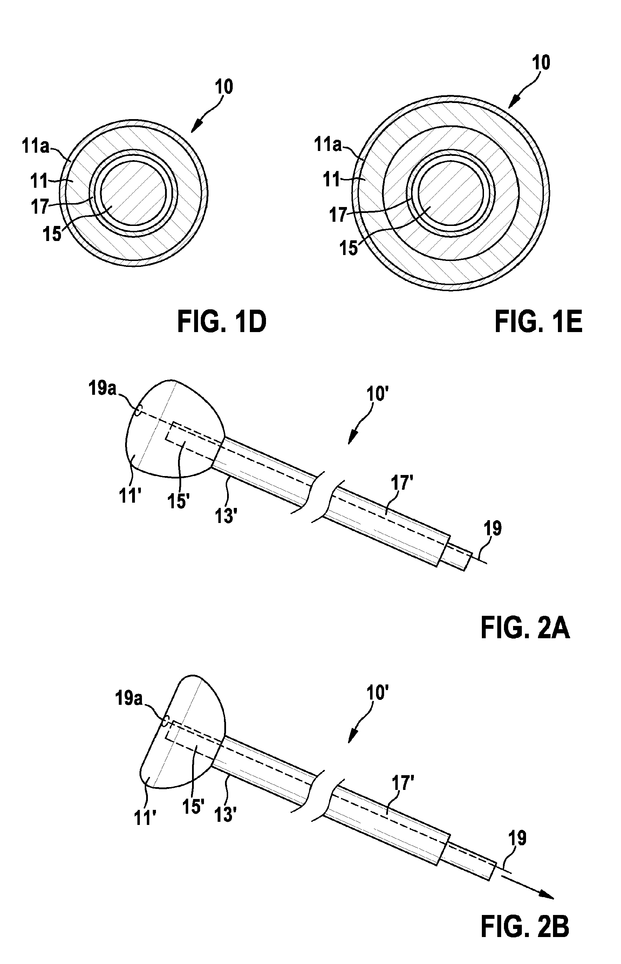

Fig. 1A bis 1E zeigen schematisch einen Katheter 10 mit einem distalen Ende 10d und einem proximalen Ende 10p, bei dem am distalen Ende ein Deformationskörper 11 angebracht ist. In der dargestellten Ausführung handelt es sich um einen Schaumstoffkörper mit einer formelastischen Hülle 11 a, die sich bei Wandkontakt des distalen Katheterendes 10d, wie Fig. 1C zeigt, bauchig verformt.

Ein Katheterkörper 13 des Katheters 10 umfasst einen Innenschlauch 15 und einen diesem gegenüber proximal verschieblichen Außenschlauch 17. Am distalen Ende des Innenschlauches ist der Deformationskörper 11 befestigt, und der Außenschlauch überdeckt in einem Ausgangszustand des Katheters (Fig. 1A) vollständig sowohl den Innenschlauch als auch den diesen im distalen Bereich umgebenden Deformationskörper und lässt letzteren sich erst entfalten, wenn er hinreichend weit zurückgezogen ist (Fig. 1B). Aufgrund der Verschieblichkeit von Innen- und Außenschlauch relativ zueinander führt, wie in Fig. 1C zu erkennen, die Einwirkung einer Druckkraft F auf das distale Ende des Katheters neben der Deformation des Ballons 11 zudem zu einer Verschiebung des Innenschlauches 15 relativ zum Außenschlauch 17 in proximaler Richtung. Diese Verschiebung kann detektiert und ausgewertet werden; zum messtechnischen Einsatz des vorgeschlagenen Katheters siehe im Übrigen die Ausführungen weiter unten.

Fig. 2A und 2B zeigen einen weiteren erfindungsgemäßen Katheter, wobei an Fig. 1A bis 1D angelehnte Bezugsziffern gewählt sind und weiter oben im Bezug auf die erste Ausführung gegebene Erläuterungen hier nicht wiederholt werden.

2A zeigt einen Ausgangszustand und Fig. 2B einen Gebrauchszustand unter Einwirkung einer distal wirkenden Kraft F. Der Katheter 10' unterscheidet sich von dem in Fig. 1A bis 1E gezeigten und weiter oben beschriebenen dadurch, dass der Deformationskörper 11' über das distale Ende des Innenschlauches 15' hinausragt. Ein Wandkontakt des Katheters mit der Wandung eines Gefäßes oder Hohlorgans in distaler Richtung führt also zuerst zu einem Stauchen des Deformationskörpers, wie in Fig. 2B zu erkennen ist. Erst weiteres Vorschieben des Katheters führt (neben einer weiteren Ausprägung dieser Stauchung bzw. Verformung) letztlich zu einem hinreichend hohen Druck auf das distale Ende des Innenschlauches 15, dass dieser sich nach proximal verschiebt. Der Katheter kann allerdings auch so aufgebaut sein, dass - nach der Freigabe des Deformationskörpers durch Zurückziehen des Außenschlauches - der Innenschlauch gegenüber dem Außenschlauch verriegelt ist und nicht nach proximal ausweichen kann.

Wie bei der erstgenannten Ausführung wird durch die Verschiebung in Verbindung mit der Deformation des Deformationskörpers die Flächenpressung am distalen Katheterende und somit auch eine Perforationsgefahr vermindert. Das Vorsehen eines Deformations-Messdrahtes 19, der durch die gesamte Länge des Innenschlauches 15' des Katheters 10' verläuft und über ein Fixierungs-Pad 19a an dem am meisten distal gelegenen Punkt des Deformationskörpers 11' fixiert ist, ermöglicht zudem eine näherungsweise Erfassung der bei einem Wandkontakt erfolgenden Deformation des Deformationskörpers. Er verschiebt sich nämlich im Umfang der stattfindenden Deformation nach proximal, wie in Fig. 2B durch den Pfeil am proximalen Ende symbolisch dargestellt ist.

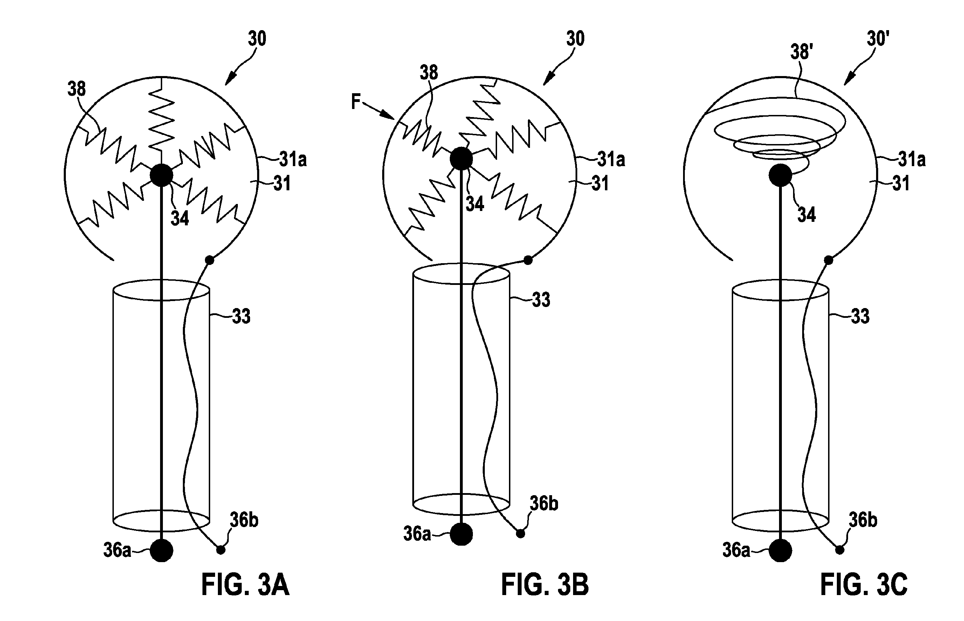

Fig. 3A und 3B zeigen schematisch das Funktionsprinzip einer weiteren Ausführung des vorgeschlagenen Katheters, nämlich einen Katheter 30 mit einem Deformationskörper 31 am distalen Ende eines (im Kontext der nachstehend beschriebenen Funktion) als starr anzusehenden Katheterkörpers 33. Der Katheter 30 umfasst eine in seiner Mittenachse liegenden ersten Pol 34 einer elektrischen (beispielsweise induktiven) Messeinrichtung, deren zweiter Pol durch die (insbesondere leitfähig ausgestaltete) Hülle 31a des Deformationskörpers gebildet ist. Beide Messpole 34, 31a sind jeweils mit einem proximalen Messanschluss 36a, 36b des Katheters verbunden. Die (leitfähige) Hülle bzw. Mantelschicht 31a ist gegenüber dem zentral fixierten ersten Messpol 34 durch eine Mehrzahl von Federelementen 38 elastisch und selbst-zentrierend verspannt. Die Federelemente 38 sind hier zur Verdeutlichung des Funktionsprinzips als Federn gezeichnet, in der Praxis kann es sich um Deformationselemente anderen Aufbaus handeln, wobei im Kontext der oben angesprochenen induktiven Messung eine Funktion als Induktivitäten zweckmäßig sein kann.

Wie in Fig. 3B zu erkennen, verschiebt sich unter dem Einfluss einer Krafteinwirkung F die Konfiguration aus Federelementen 38 und Hülle 31a des Deformationskörpers 31, was zur Erzeugung eines elektrischen Messsignals an den Anschlüssen 36a, 36b führt. Dieses Signal korreliert mit dem Verschiebungsbetrag und/oder Deformationsbetrag des Deformationskörpers und ist somit als Maß für die einwirkende Wandkraft brauchbar. Die Abhängigkeit des Messsignals von der Verformung wird z. B. einer vorab bestimmten Look-up-Tabelle entnommen. Je nach konkreter Ausgestaltung kann eine Anordnung der gezeigten Art als induktiver oder kapazitiver oder auch als ohmscher Messwertaufnehmer funktionieren oder ggfs. eine Kombination mehrerer Messprinzipien realisieren.

Fig. 3C zeigt einen modifizierten Katheter 30', dessen Aufbau im Wesentlichen mit demjenigen des Katheters 30 nach Fig. 3A und 3B übereinstimmt, bei dem jedoch die Anordnung aus mehreren Federelementen durch ein einzelnes, speziell spiralig geformtes Federelement 38' ersetzt ist. Hinsichtlich der realisierbaren Messprinzipien gelten auch für diese Modifikation grundsätzlich die vorstehenden Feststellungen.

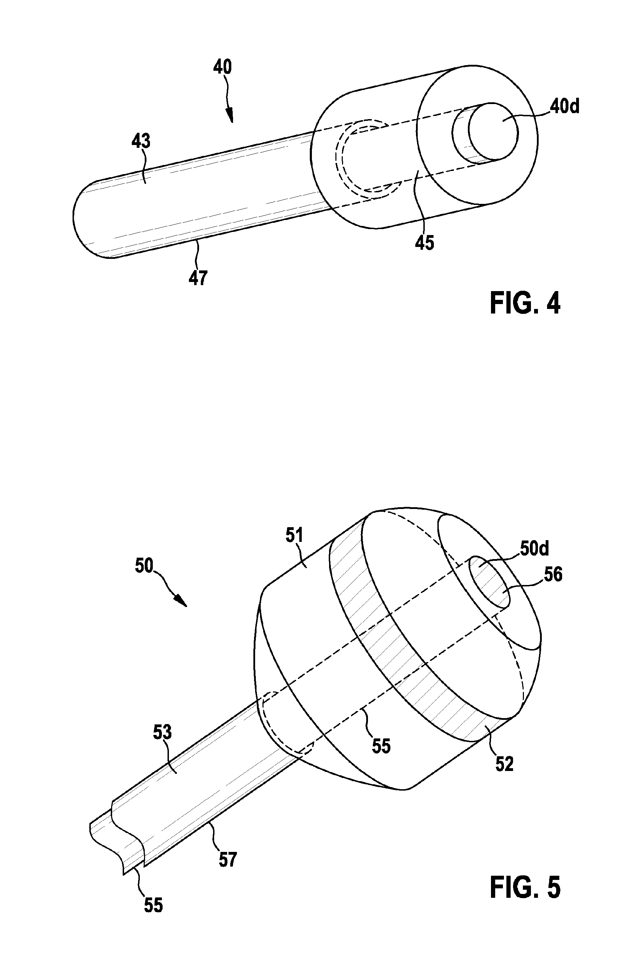

Fig. 4 zeigt als weitere Ausführung der Erfindung schematisch einen Drei-Abschnitts-Katheter 40, bei dem sowohl der Katheterkörper 43 (einschließlich Innenschlauch 45 und Außenschlauch 47) als auch der Deformationskörper 41 in drei Zylindersegment-Abschnitte unterteilt ist. Bekommt das Katheter 40 nahe seinem distalen Ende 40d im Gebrauch Wandkontakt an der Wandung eines Gefäßes oder Hohlorgans, so wirkt sich dieser Wandkontakt für die einzelnen Abschnitte des Deformationskörpers unterschiedlich aus, und es kann etwa über die Erfassung des elektrischen Widerstandes der einzelnen Teile neben der Gesamtkraft auch die Richtung bestimmt und damit eine zusätzliche Kenntnis der Lage des Katheters gewonnen werden.

Fig. 5 zeigt schematisch das distale Ende eines erfindungsgemäßen Elektrodenkatheters 50 mit einem Deformationskörper 51 und Katheterkörper 53, der einen Innenschlauch 55 und Außenschlauch 57 umfasst und in seinem distalen Bereich zwei Elektroden trägt, die zur Gewebestimulation und/oder zum Abfühlen von Gewebepotentialen eingesetzt werden können. Am distalen Ende 50d des Elektrodenkatheters ist eine Spitzenelektrode 56 vorgesehen, die beispielsweise durch eine Metallbeschichtung des distalen Endes des Innenschlauches 55 erzeugt sein kann. Daneben ist auf dem Umfang des Deformationskörpers 51 eine Ringelektrode 52 vorgesehen, die etwa aus einem dehnbaren leitfähigen Kunststoff gebildet sein kann. Alternativ ist auch beispielsweise das Vorsehen eines mäanderförmigen Metallstreifens o. ä. möglich.

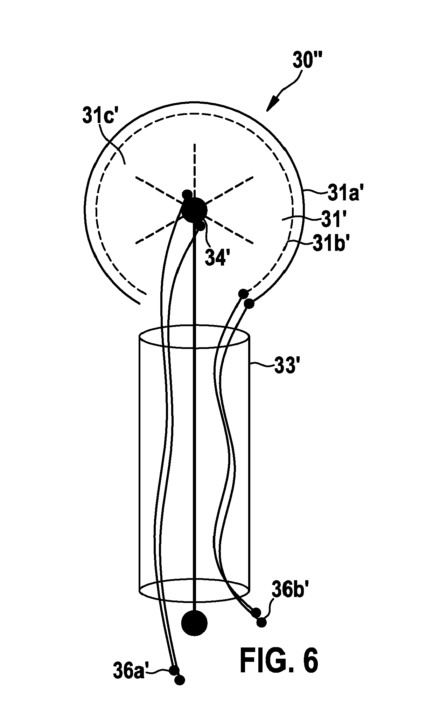

Fig. 6 zeigt schematisch einen weiteren erfindungsgemäßen Katheter, wobei die Bezeichnung der Teile an diejenige des Katheters 30 nach Fig. 3A und 3B angelehnt ist. Der Katheter insgesamt mit der Ziffer 30" bezeichnet und umfasst einen Deformationskörper 31' und einen als starr anzusehenden Katheterkörper 33', wobei in Achsenlage und positionsfest zum Katheterkörper 33' ein erstes Messeinrichtungselement 34' und an der Innenseite der Hülle 31a des Deformationskörpers ein zweites Messeinrichtungs-Element 31b' vorgesehen ist.

Abweichend von der Ausführung nach Fig. 3A und 3B, handelt es sich bei der Messeinrichtung hier um eine optische Messeinrichtung und beim ersten Messeinrichtungs-Element 34' um eine gleichmäßig rundum strahlende Lichtquelle und beim zweiten Messeinrichtungs-Element 31b' um ein Kugel-Array aus Solarzellen. Der Deformationskörper ist mit einem Fluid mit lichtdämpfender Wirkung, etwa einem trüben Elastogel, 3 1 c' gefüllt. Die Lichtquelle 34' ist durch erste proximale Anschlüsse 36a' mit einer externen Stormversorgung verbunden, und das Solarzellen-Array 31b' ist über zweite proximale Anschlüsse 36b' mit einer externen Mess- und Auswertungseinrichtung (nicht dargestellt) verbunden.

Die Lichtquelle 34' strahlt durch das lichtdämpfende Medium 31c', und die ankommende Strahlung wird durch das Kugel-Array von Solarzellen 31b' aufintegriert. Das Kugel-Array von Solarzellen auf der Innenoberfläche der Hülle 31 kann beispielsweise mittels eines Druckverfahrens erzeugt werden, welches zur Erzeugung von Solarzellen neuerdings in Betracht gezogen wird. Der Integralwert verändert sich, wenn die Lichtanteile wegen einer Verschiebung der Quelle aus der Mitte des Deformationskörpers (bedingt durch Krafteinwirkungen auf diesen) unterschiedlich lange Wege durch das lichtdämpfende Medium zurücklegen müssen und daher in anderer Weise gedämpft werden als im ungestörten Ruhezustand des Deformationskörpers.

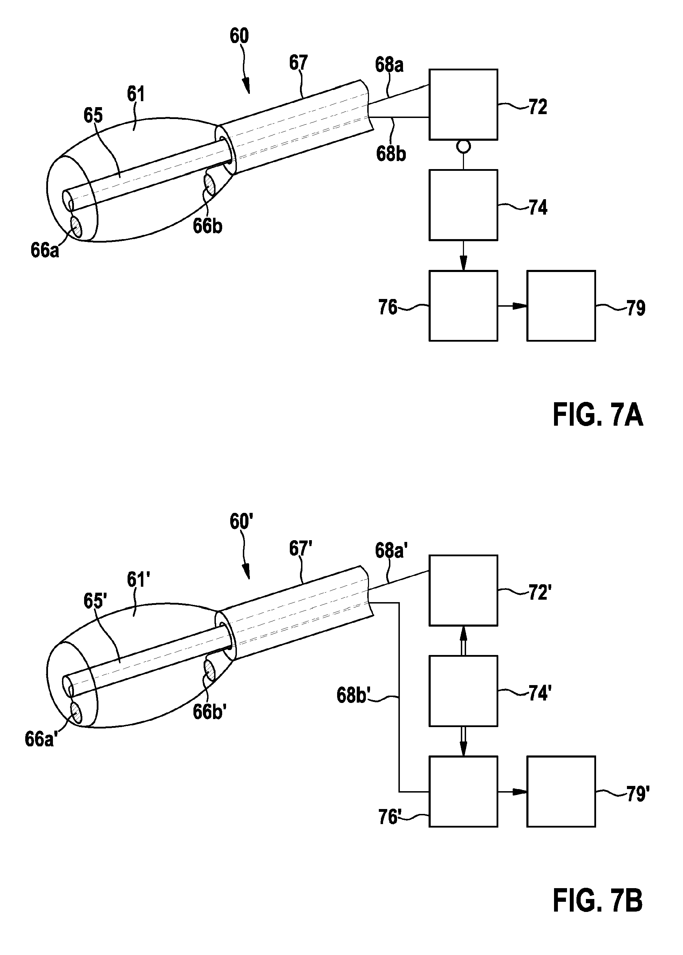

Fig. 7A zeigt eine Katheteranordnung mit einem Katheter 60, welcher als Deformationskörper einen Schaumstoffkörper 61 aus leitfähigem Schaum aufweist.

Eine Erfassung der Deformation des Deformationskörpers 61, der einen durch richtungsabhängige Impedanzänderungen auf Verformungsgrad und -richtung ansprechenden leitfähigen Schaum aufweist, erfolgt über zwei Messelektrodenflächen 66a, 66b im distalen bzw. proximalen Bereich des Deformationskörpers. Diese sind über Messleitungen 68a bzw. 68b an eine Messstromversorgung 72 mit zugeordnetem Stromfühler 74 angeschlossen. Dem Stromfühler 74 sind eine Auswertungseinheit 76 sowie schließlich eine Anzeigeeinheit 79 zur Bereitstellung von Wandkontakt-Informationen für den Operateur nachgeschaltet. Eine Deformation des Deformationskörpers 61 führt zu einer Verringung des Abstandes zwischen den Messelektroden 66a, 66b und zugleich einer Komprimierung des Schaums, die sich ein einer Änderung des Widerstands im Strompfad zwischen den Messelektroden und somit in einer Änderung der Stromstärke ausdrückt. Deren Auswertung liefert die benötigte Information über das Bestehen eines Wandkontaktes und dessen Intensität.

Ähnlich aufgebaut ist die in Fig. 7B schematisch gezeigte Ausführung mit einem Katheter 60', dessen Deformationskörper 61' eine in gewissem Grade transparente Schaumstoffzusammensetzung aufweist. Dieser ermöglicht die Erfassung der Schwächung von in Längsrichtung durch den Deformationskörper durchgehendem Licht bei einer Stauchung desselben (wie in Fig. 2B skizziert). Anstelle von Messelektroden sind dementsprechend hier ein optisches Sendeelement (z.B. eine LED) 66a' und ein optisches Empfangselement (eine Fotodiode) 66b' vorgesehen, die über elektrische Zuleitungen an eine entsprechend angepasste Messstromversorgung 72' bzw. Auswertungseinheit 76' angeschlossen sind, welche über eine Betriebssteuereinheit 74' gesteuert werden. Zur Anordnung gehört auch wiederum eine Anzeigeeinheit 79', auf der die Messergebnisse für den Operateur visualisiert werden.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Katheter mit einem langgestreckten Katheterkörper, der in Bezug auf eine Gebrauchslage ein distales und ein proximales Ende hat, wobei am distalen Ende ein schwamm- oder kissenartiger elastischer Deformationskörper angeordnet ist, welcher, insbesondere elektrisch, mechanisch oder optisch wirkende, Messmittel oder einen Messanschluss zur Erfassung einer auf ihn ausgeübten Andruckkraft aufweist.

2. Katheter nach Anspruch 1, wobei der Deformationskörper einen Kunststoffschaum oder ein Fluid, insbesondere eine Flüssigkeit oder ein Gel, oder eine Füllung aus schüttfähigen Partikeln, enthält.

3. Katheter nach Anspruch 1 oder 2, wobei in einander gegenüberliegenden Mantelabschnitten des Deformationskörpers Messelektroden mit jeweils einem Messspannungsanschluss vorgesehen sind.

4. Katheter nach Anspruch 3, wobei der Deformationskörper einen Schaum aus einem elektrisch leitfähigen Polymeren oder mit elektrisch leitfähigen, fein verteilten Einlagerungen oder mit leitfähigen Partikeln, die mit einem Dielektrikum beschichtet sind, oder Teilchen aus einer feroelektrischen Folie aufweist.

5. Katheter nach Anspruch 1 oder 2, wobei in einander gegenüberliegenden Mantelabschnitten des Deformationskörpers ein optisches Sende- und Empfangselement mit einem Stromversorgungs- bzw. Messanschluss vorgesehen sind.

6. Katheter nach Anspruch 5, wobei der Deformationskörper fein verteilte lichtreflektierende Einlagerungen und/oder eine innenseitig lichtreflektierende Hülle oder Mantelschicht aufweist.

7. Katheter nach einem der vorangehenden Ansprüche, wobei am distalen Ende des Katheterkörpers, starr mit diesem verbunden, ein erstes Messvorrichtungselement und auf der Innenwandung einer Hülle des Deformationskörpers ein zweites, mit dem ersten zusammenwirkendes Messvorrichtungselement vorgesehen ist und die Hülle des Deformationskörpers durch Ihre eigene Elastizität und/oder ihr Zusammenwirken mit einer Füllung und/oder ihr Zusammenwirken mit mindestens einem Federelement, welches die Hülle gegenüber dem Katheterkörper abstützt, bezüglich des Katheterkörpers selbst-rückstellend ausgeführt ist.

8. Katheter nach Anspruch 1 oder 2, wobei an einem distalen Ende des Deformationskörpers ein sich bis zum proximalen Ende des Katheters erstreckender, im Katheterkörper verlaufender und frei verschieblicher Messdraht angebracht ist.

9. Katheter nach einem der vorangehenden Ansprüche, wobei der Deformationskörper eine formelastische Hülle oder Mantelschicht aufweist.

10. Katheter nach einem der vorangehenden Ansprüche, wobei der Deformationskörper mehrteilig aus mehreren Teilkörpern ausgebildet ist, wobei die Teilkörper unterschiedliches Deformationsverhalten und/oder separate Messmittel oder -anschlüsse zur Erfassung einer spezifisch auf sie ausgeübten Andruckkraft aufweisen.

11. Katheter nach Anspruch 10, wobei die Teilkörper optisch und/oder elektrisch wirkende Messmittel oder -anschlüsse aufweisen.

12. Katheter nach einem der vorangehenden Ansprüche, ausgebildet als Elektrodenleitung mit mindestens einer auf dem Deformationskörper angeordneten Stimulations- und/oder Abfühlelektrode.

13. Katheter nach Anspruch 12, wobei die oder mindestens eine Stimulations- und/oder Abfühlelektrode elastisch deformierbar, insbesondere aus leitfähigem Kunststoff gebildet, ist.

14. Katheter nach einem der vorangehenden Ansprüche, wobei der Deformationskörper im entspannten, unverformten Zustand Kugel- oder Ellipsoid- oder Zylinderform aufweist.

15. Katheteranordnung mit einem Katheter nach einem der vorangehenden Ansprüche und einer mit dem oder jedem Messmittel oder -anschluss verbundenen Messeinrichtung zur Bestimmung einer auf den Deformationskörper oder Teilbereiche desselben einwirkenden Andruckkraft.
